# EUROPEAN PATENT APPLICATION

(11) **EP 3 806 222 A1**
(43) Date of publication of application: **14.04.2021**
(21) Application number: 19811827.5
(22) Date of filing: 21.05.2019
(51) Int. Cl.: H01M 10/0567, H01G 11/62, H01G 11/64, H01M 10/052, H01M 10/0568

(54) **NON-AQUEOUS ELECTROLYTE FOR POWER STORAGE DEVICE, AND POWER STORAGE DEVICE**

(30) Priority: 31.05.2018 JP 2018104498
(71) Applicant: MU Ionic Solutions Corporation, Tokyo 1008251 (JP); Sumitomo Seika Chemicals Co., Ltd., Kako-gun, Hyogo 675-0145 (JP)
(72) Inventor: KONDO, Masahide, Minato-ku, Tokyo 105-8449 (JP); SHIMAMOTO, Kei, Sakai-shi, Osaka 592-8543 (JP)
(74) Representative: Hoffmann Eitle
(86) International application number: PCT/JP2019/020162
(87) International publication number: WO 2019/230506

(57) **Abstract**

Disclosed is a non-aqueous electrolytic solution for an energy storage device, including a non-aqueous solvent, an electrolyte salt, a first additive consisting of a sulfolane compound represented by the following formula (1), and a second additive which is at least one selected from the group consisting of a cyclic sulfuric ester compound represented by the following formula (2a) and a dinitrile compound represented by the following formula (2b):

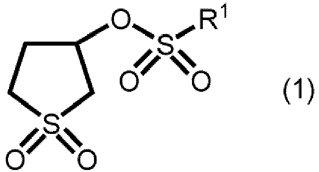

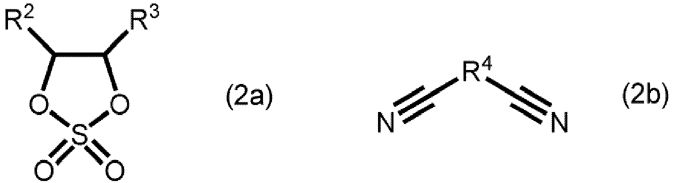

## Description

### Technical Field

The present invention relates to a non-aqueous electrolytic solution for an energy storage device, and an energy storage device.

### Background Art

An energy storage device using a non-aqueous electrolytic solution, typified by a lithium secondary battery, has been widely used as a power source for small electronic devices, a power source for electric vehicles or electric power storage, or the like. In order to improve the durability of the energy storage device, and the like, various additives such as a sulfonate compound may be added to a non-aqueous electrolytic solution for an energy storage device in some cases (for example, Patent Literature 1).

### Citation List

### Patent Literature

Patent Literature 1: U.S. Unexamined Patent Publication No. 2017/0271715

### Summary of Invention

### Technical Problem

Battery characteristics such as a capacity and a resistance of an energy storage device may be significantly reduced in a high-temperature environment in some cases. In addition, due to generation of gas from a sealed non-aqueous electrolytic solution in a high-temperature environment, the power storage device may be expanded, resulting in hindering a use thereof in some cases.

Therefore, an object of an aspect of the present invention is to provide a non-aqueous electrolytic solution that can provide an energy storage device having excellent capacity stability while suppressing an increase in a resistance and gas generation in a high-temperature environment.

### Solution to Problem

An aspect of the present invention relates to a non-aqueous electrolytic solution for an energy storage device, including a non-aqueous solvent, an electrolyte salt dissolved in the non-aqueous solvent, a first additive consisting of a sulfolane compound represented by the following formula (1), and a second additive that is at least one selected from the group consisting of a cyclic sulfuric ester compound represented by the following formula (2a) and a dinitrile compound represented by the following formula (2b) (a second additive consisting of at least one of a cyclic sulfuric ester compound represented by the following formula (2a) or a dinitrile compound represented by the following formula (2b)).

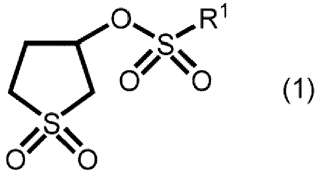

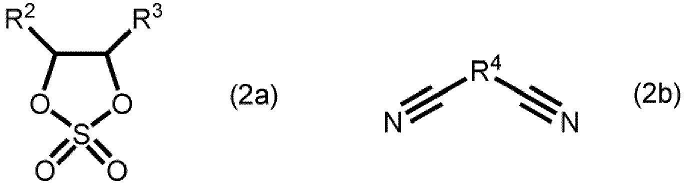

In the formula (1), R¹ represents a methyl group, an ethyl group, a vinyl group, a phenyl group, or a tolyl group. In the formula (2a), R² and R³ each independently represent a methyl group, an ethyl group, or a hydrogen atom, and in the formula (2b), R⁴ represents an alkylene group having 1 to 6 carbon atoms.

Another aspect of the present invention relates to an energy storage device including the non-aqueous electrolytic solution for an energy storage device, a positive electrode, and a negative electrode.

### Advantageous Effects of Invention

According to the present invention, a non-aqueous electrolytic solution that can provide an energy storage device having excellent capacity stability in a high-temperature environment while suppressing an increase in a resistance and gas generation is provided.

### Brief Description of Drawings

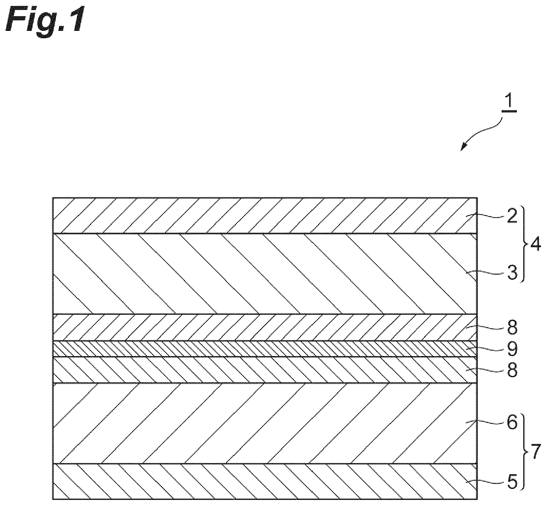

FIG. 1 is a cross-sectional view illustrating an embodiment of an energy storage device.

### Description of Embodiments

Hereinafter, some embodiments of the present invention will be described in detail. However, the present invention is not limited to the following embodiments.

A non-aqueous electrolytic solution according to an embodiment includes a non-aqueous solvent, an electrolyte salt dissolved in the non-aqueous solvent, a first additive consisting of a sulfolane compound represented by the following formula (1), and a second additive that is at least one selected from the group consisting of a cyclic sulfuric ester compound represented by the following formula (2a) and a dinitrile compound represented by the following formula (2b):

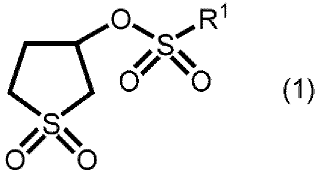

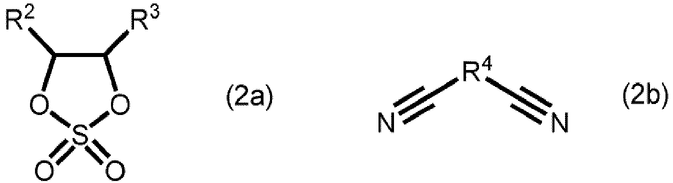

### First Additive

The first additive is consisting of one or two or more sulfolane compounds represented by the formula (1). In the formula (1), R¹ represents a methyl group, an ethyl group, a vinyl group, a phenyl group, or a tolyl group.

A content of the first additive in the non-aqueous electrolytic solution may be 0.1% by mass or more, or 0.3% by mass or more, and may be 5% by mass or less, or 2% by mass or less, based on the mass of the non-aqueous electrolytic solution. When the content of the first additive is within these ranges, an energy storage device having further excellent storage stability in a high-temperature environment is easily obtained.

### Second Additive

The second additive is consisting of at least one of the cyclic sulfuric ester compound represented by the formula (2a) and the dinitrile compound represented by the formula (2b). In the formula (2a), R² and R³ each independently represent a methyl group, an ethyl group, or a hydrogen atom. In the formula (2b), R⁴ represents an alkylene group having 1 to 6 carbon atoms. R⁴ may be a linear alkylene group such as a methanediyl group, an ethanediyl group, a propanediyl group, a butanediyl group, a pentanediyl group, and a hexanediyl group.

A content of the second additive in the non-aqueous electrolytic solution may be 0.1% by mass or more, or 0.3% by mass or more, and may be 5% by mass or less, or 2% by mass or less, based on the mass of the non-aqueous electrolytic solution. When the content of the second additive is within these ranges, an energy storage device having further excellent storage stability in a high-temperature environment is easily obtained. From the same viewpoint, a mass ratio of the content of the first additive to the content of the second additive may be 50 : 50 to 95 : 5.

### Electrolyte Salt

The electrolyte salt included in the non-aqueous electrolytic solution according to an embodiment is typically a lithium salt. Examples of the lithium salt which can be used as the electrolyte salt include inorganic lithium salts such as lithium hexafluorophosphate (LiPF₆) and lithium tetrafluoroborate (LiBF₄) and imide salts such as LiN(SO₂F)₂(LiFSI) and LiN(SO₂CF₃)₂. Only one kind or a combination of two or more kinds thereof can be used. As the electrolyte salt, a compound different from the lithium salt as a third additive which will be described later is used.

A concentration of the electrolyte salt may be 0.3 M or more, 0.7 M or more, or 1.1 M or more, and may be 2.5 M or less, 2 M or less, or 1.6 M or less, based on the volume of the non-aqueous electrolytic solution.

### Non-Aqueous Solvent

The non-aqueous solvent included in the non-aqueous electrolytic solution according to an embodiment may include one or two or more selected from the group consisting of, for example, a cyclic carbonate, a chain carbonate, a chain carboxylic ester, a lactone, an ether, and an amide.

Examples of the cyclic carbonate include ethylene carbonate (EC), propylene carbonate (PC), 1,2-butylene carbonate, 2,3-butylene carbonate, 4-fluoro-1,3-dioxolan-2-one (FEC), trans- or cis-4,5-difluoro-1,3-dioxolan-2-one (the both are hereinafter generically referred to as "DFEC"), vinylene carbonate (VC), vinyl ethylene carbonate (VEC), and 4-ethynyl-1,3-dioxolan-2-one (EEC).

Examples of the chain carbonate include asymmetric chain carbonates such as methyl ethyl carbonate (MEC), methyl propyl carbonate (MPC), methyl isopropyl carbonate (MIPC), methyl butyl carbonate, and ethyl propyl carbonate; and symmetric chain carbonates such as dimethyl carbonate (DMC), diethyl carbonate (DEC), dipropyl carbonate, and dibutyl carbonate.

Examples of the chain carboxylic ester include methyl pivalate, ethyl pivalate, propyl pivalate, methyl propionate, ethyl propionate (EP), propyl propionate, methyl acetate, and ethyl acetate (EA).

Examples of the lactone include γ-butyrolactone (GBL), γ-valerolactone, and α-angelica lactone.

Examples of the ether include cyclic ethers such as tetrahydrofuran, 2-methyltetrahydrofuran, and 1,4-dioxane; and chain ethers such as 1,2-dimethoxyethane, 1,2-diethoxyethane, and 1,2-dibutoxyethane.

Examples of the sulfone include sulfolane.

The non-aqueous solvent may include at least one specific solvent selected from vinylene carbonate, fluoroethylene carbonate, and 1,3-propanesultone, and another solvent. In this case, a content of the specific solvent such as vinylene carbonate selected from the above-mentioned solvents may be 0.1 to 5% by mass with respect to the total mass of the non-aqueous electrolytic solution.

### Other Components

The non-aqueous electrolytic solution according to an embodiment may further contain a third additive formed of at least one lithium salt represented by the following formula (3a), (3b), (3c), (3d), or (3e). Thus, an energy storage device having further excellent storage stability in a high-temperature environment is easily obtained.

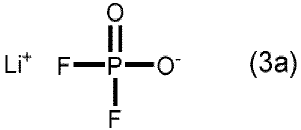

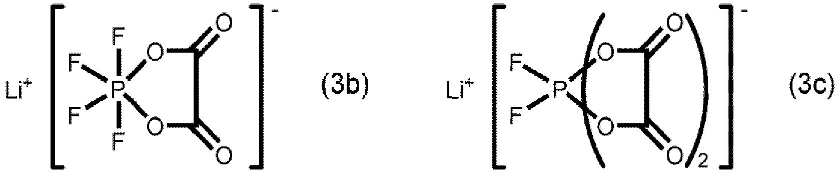

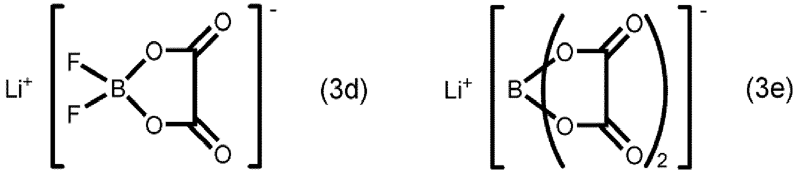

A content of the third additive in the non-aqueous electrolytic solution may be 0.1% by mass or more, or 0.2% by mass or more, and may be 2% by mass or less, or 1.5% by mass or less, based on the mass of the non-aqueous electrolytic solution. When the content of the third additive is within these ranges, an energy storage device having further excellent storage stability in a high-temperature environment is easily obtained.

The non-aqueous electrolytic solution according to an embodiment can further contain other components such as additives other than those exemplified above, if necessary. The non-aqueous electrolytic solution can be prepared by mixing the respective components.

### Power Storage Device

FIG. 1 is a cross-sectional view schematically illustrating one embodiment of an energy storage device. An energy storage device 1 illustrated in FIG. 1 is a non-aqueous electrolytic solution secondary battery. The power storage device 1 includes a plate-shaped positive electrode 4, a plate-shaped negative electrode 7 facing the positive electrode 4, a non-aqueous electrolytic solution 8 disposed between the positive electrode 4 and the negative electrode 7, and a separator 9 provided in the non-aqueous electrolytic solution 8. The positive electrode 4 has a positive electrode current collector 2 and a positive electrode active material layer 3 provided on the non-aqueous electrolytic solution 8 side. The negative electrode 7 has a negative electrode current collector 5 and a negative electrode active material layer 6 provided on the non-aqueous electrolytic solution 8 side. As the non-aqueous electrolytic solution 8, the non-aqueous electrolytic solution according to the above-mentioned embodiment can be used. Although the non-aqueous electrolytic solution secondary battery is illustrated as an energy storage device in FIG. 1, an energy storage device obtained by applying the non-aqueous electrolytic solution is not limited thereto, and may be another power storage device such as an electric double layer capacitor.

The positive electrode current collector 2 and the negative electrode current collector 5 may be, for example, a metal foil made of a metal such as aluminum, copper, nickel, and stainless steel.

The positive electrode active material layer 3 includes a positive electrode active material. The positive electrode active material may be, for example, a complex metal oxide of lithium, which contains one or two or more selected from the group consisting of cobalt, manganese, and nickel. As the positive electrode active material, one or two or more of the complex metal oxides of lithium can be used. Examples of the complex metal oxide of lithium include LiCoO₂, LiCo₁₋ₓMₓO₂ (in which M is one or two or more elements selected from the group consisting of Sn, Mg, Fe, Ti, Al, Zr, Cr, V, Ga, Zn, and Cu, 0.001 ≤ x ≤ 0.05), LiMn₂O₄, LiNiO₂, LiCo₁₋ₓNiₓO₂ (0.01 < x < 1), LiCo_{1/3}Ni_{1/3}Mn_{1/3}O₂, LiNi_{0.5}Mn_{0.3}Co_{0.2}O₂, LiNi_{0.6}Mn_{0.2}Co_{0.2}O₂, LiNi_{0.8}Mn_{0.1}Co_{0.1}O₂, LiNi_{0.8}Co_{0.15}Al_{0.05}O₂, a solid solution of Li₂MnO₃ and LiMO₂ (in which M is a transition metal such as Co, Ni, Mn, and Fe), and LiNi_{1/2}Mn_{3/2}O₄.

Other examples of the positive electrode active material include lithium-containing olivine-type phosphate. The lithium-containing olivine-type phosphate may contain one or two or more selected from the group consisting of iron, cobalt, nickel, and manganese. Specific examples of the lithium-containing olivine-type phosphate include LiFePO₄, LiCoPO₄, LiNiPO₄, LiMnPO₄, and LiFe₁₋ₓMnₓPO₄ (0.1 < x < 0.9). These lithium-containing olivine-type phosphates may be partially substituted with other elements.

The positive electrode active material layer 3 may further include a conductive agent. Examples of the conductive agent include graphite such as natural graphite and artificial graphite, and carbon black such as acetylene black, Ketjen black, channel black, furnace black, lamp black, and thermal black.

The positive electrode active material layer 3 may further include a binder. Examples of the binder include polytetrafluoroethylene (PTFE), polyvinylidene fluoride (PVDF), a copolymer (SBR) of styrene and butadiene, a copolymer (NBR) of acrylonitrile and butadiene, carboxymethyl cellulose (CMC), and an ethylene-propylene-diene terpolymer.

The negative electrode active material layer 6 includes a negative electrode active material. The negative electrode active material may be any of a lithium metal, a lithium alloy, or a material capable of absorbing and releasing lithium ions, and examples thereof include a carbon material (for example, artificial graphite and natural graphite), an elemental metal (including an alloy thereof), or a metal compound. The negative electrode active material may also be an element of a metal such as Si, Ge, Sn, Pb, P, Sb, Bi, Al, Ga, In, Ti, Mn, Fe, Co, Ni, Cu, Zn, Ag, Mg, Sr, and Ba, or a metal compound containing at least one metal selected from these metals. An alloy of the metal and lithium may also be used as the negative electrode active material. The metal compound may be, for example, an oxide, a nitride, a sulfide, or a boride.

The separator 9 can be a single-layer or multi-layer film. The separator is not particularly limited, but may be a microporous film formed of a single layer or laminate of a polyolefin such as polypropylene, polyethylene, and an ethylene-propylene copolymer, a woven fabric, a nonwoven fabric, or the like. The separator may be a laminate of polyethylene and polypropylene or a laminate having a three-layer structure of polypropylene/polyethylene/polypropylene.

The thickness of the separator 9 is not particularly limited, but may be 2 µm or more, 3 µm or more, or 4 µm or more, and may be 30 µm or less, 20 µm or less, or 15 µm or less.

A configuration of the power storage device is not limited to the embodiment of FIG. 1. Specific forms such as a shape and a thickness of each member which constitutes the power storage device can be appropriately set by those skilled in the art. For example, the power storage device may be a coin-type battery, a cylinder-type battery, a prismatic battery, or a laminate-type battery.

### Examples

Hereinafter, the present invention will be more specifically described with reference to Examples. However, the present invention is not limited to these Examples.

### 1. Preparation of Non-Aqueous Electrolytic Solution

The following compound 1-1 was prepared as a first additive and the following 2a-1 was prepared as a second additive.

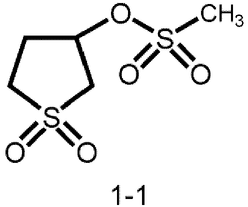

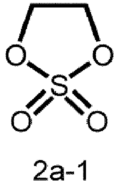

### Example 1

A non-aqueous electrolytic solution that included a non-aqueous solvent including ethylene carbonate (EC), methyl ethyl carbonate (MEC), and dimethyl carbonate (DMC) at a volume ratio of EC/MEC/DMC = 3/3/4, lithium hexafluorophosphate as an electrolyte salt (LiPF₆, concentration of 1.2 M), a compound 1-1 (content: 1% by mass), and a compound 2a-1 (content: 1% by mass) was prepared.

### Example 2

A non-aqueous electrolytic solution was obtained in the same manner as in Example 1, except that the contents of the compound 1 and the compound 2a-1 were changed to 0.5% by mass , respectively.

### Comparative Examples 1 to 3

A non-aqueous electrolytic solution was obtained in the same manner as in Example 1, except that the type and the concentration of each component were changed as shown in Table 1.

**Table 1**

| | | Ex. 1 | Ex. 2 | Comp. Ex. 1 | Comp. Ex. 2 | Comp. Ex. 3 |
|---|---|---|---|---|---|---|
| Electrolyte salt | | LiPF₆ (1.2 M) | LiPF₆ (1.2 M) | LiPF₆ (1.2 M) | LiPF₆ (1.2 M) | LiPF₆ (1.2 M) |
| First additive Sulfolane compound | | Cpd. 1-1 (1 wt.%) | Cpd. 1-1 (0.5 wt.%) | - | Cpd. 1-1 (1 wt.%) | - |
| Second additive Cyclic sulfuric ester | | Cpd. 2a-1 (1 wt.%) | Cpd. 2a-1 (0.5 wt.%) | - | - | Cpd. 2a-1 (1 wt.%) |
| Battery characteristics (relative values in case of value in Comparative Example 1 being taken as 100) | Discharge capacity | 116 | 110 | 100 | 108 | 115 |
| | DCIR | 67 | 69 | 100 | 72 | 79 |
| | Amount of gas generated | 34 | 49 | 100 | 66 | 55 |

### 2. Fabrication of Lithium Secondary Battery

A positive electrode mixture paste was prepared by mixing 94% by mass of LiNi_{0.5}Mn_{0.3}Co_{0.2}O₂ (positive electrode active material) and 3% by mass of acetylene black (conductive agent), and adding the mixture to a solution in which polyvinylidene fluoride (binder) was dissolved in 1-methyl-2-pyrrolidone at a concentration of 3% by mass. This positive electrode mixture paste was applied onto both surfaces of an aluminum foil and the coating film was dried to form a positive electrode active material layer. A laminated sheet formed of the aluminum foil and the positive electrode active material layer was pressed. Next, the laminated sheet was cut into a predetermined size to manufacture a strip-shaped positive electrode sheet.

A negative electrode mixture paste was prepared by adding 95% by mass of graphite (negative electrode active material) to a solution in which polyvinylidene fluoride (binder) had been dissolved in advance in 1-methyl-2-pyrrolidone at a concentration of 5% by mass. This negative electrode mixture paste was applied onto both surfaces of a copper foil and the coating film was dried to form a negative electrode active material layer. A laminated sheet formed of the copper foil and the negative electrode active material layer was pressed. Next, the laminated sheet was cut into a predetermined size to manufacture a strip-shaped negative electrode sheet.

The positive electrode sheet, the separator, and the negative electrode sheet were spirally wound in a sequentially stacked state. A microporous sheet having a three-layer structure of a polypropylene film/a polyethylene film/a polypropylene film was used as the separator. The obtained wound body was housed in a bag-shaped aluminum laminated film as an outer casing body. Next, the non-aqueous electrolytic solution of each of Examples and Comparative Examples was further injected into the outer casing body, and the outer casing body was sealed to obtain a lithium secondary battery for evaluation.

### 3. Evaluation

The lithium secondary battery was charged and discharged in one cycle in a voltage range of 2.7 V to 4.3 V at a current corresponding to 0.2 C at 25°C, then charged again to 4.3 V, and then kept at 60°C for 48 hours to perform stabilization. Then, the lithium secondary battery was discharged to 2.7 V. The lithium secondary battery after the discharge was charged to 4.3 V at a current corresponding to 0.2 at 25°C and subjected to a storage test in which the lithium secondary battery was kept for 20 days in an environment at 60°C. The discharge capacity, the DC resistance (DCIR), and the amount of gas generated after the storage test were measured by the following methods.

### Discharge Capacity

A discharge capacity when the lithium secondary battery after the storage test was discharged to 2.7 V at a current corresponding to 0.2 C at 25°C, and then charged and discharged in one cycle in a voltage range of 2.7 V to 4.3 V was measured. A ratio of the discharge capacity to a discharge capacity of Comparative Example 1 was calculated as a relative value (%) of the discharge capacity.

### DC Resistance (DCIR)

The lithium secondary battery after the storage test was charged to a charge rate of 50% at 25°C, and then a voltage was measured after 10 seconds from a start of the discharge at a current corresponding to 0.5 C, 1 C, 2 C, or 3 C. The slope of a regression line obtained when the obtained voltage values after 10 seconds were plotted against the current values was recorded as a direct current resistance. A ratio of the direct current resistance to a direct current resistance of Comparative Example 1 was calculated as a relative value (%) of the DC resistance (DCIR).

### Amount of Gas Generated

The volumes of the lithium secondary battery before and after the storage test were measured by the Archimedes method. An amount of gas generated was determined from the difference in the volumes before and after the storage test.

As shown in Table 1, it was confirmed that by using a non-aqueous electrolytic solution containing the first additive and the second additive, it is possible to obtain an energy storage device having excellent capacity stability while suppressing an increase in a resistance and gas generation when the power storage device is stored in a high-temperature environment.

### Reference Signs List

1: power storage device, 2: positive electrode current collector, 3: positive electrode active material layer, 4: positive electrode, 5: negative electrode current collector, 6: negative electrode active material layer, 7: negative electrode, 8: non-aqueous electrolytic solution, 9: separator.

## Claims

1. A non-aqueous electrolytic solution for an energy storage device, comprising:
a non-aqueous solvent;
an electrolyte salt dissolved in the non-aqueous solvent;
a first additive consisting of a sulfolane compound represented by the following formula (1); and
a second additive that is at least one selected from the group consisting of a cyclic sulfuric ester compound represented by the following formula (2a) and a dinitrile compound represented by the following formula (2b): 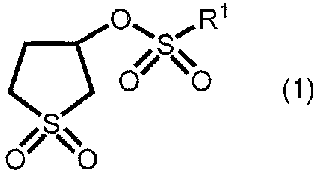 in the formula (1), R¹ represents a methyl group, an ethyl group, a vinyl group, a phenyl group, or a tolyl group, 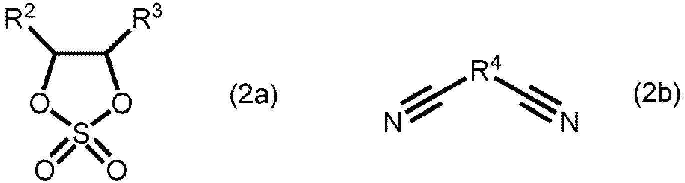
in the formula (2a), R² and R³ each independently represent a methyl group, an ethyl group, or a hydrogen atom, and
in the formula (2b), R⁴ represents an alkylene group having 1 to 6 carbon atoms.

2. The non-aqueous electrolytic solution for an energy storage device according to claim 1, further comprising a third additive formed of at least one lithium salt represented by the following formula (3a), (3b), (3c), (3d), or (3e):

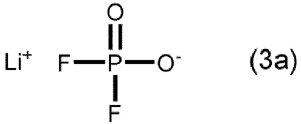

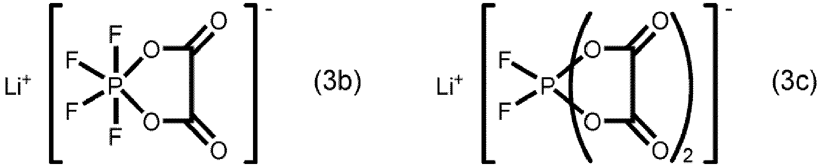

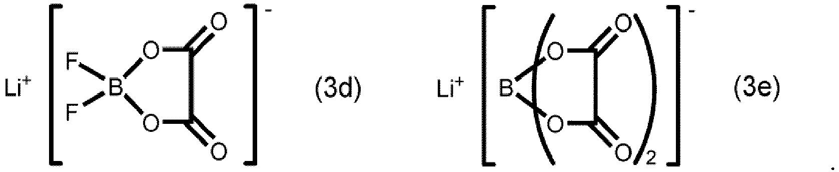

3. The non-aqueous electrolytic solution for an energy storage device according to claim 1 or 2, wherein the electrolyte salt includes at least one lithium salt selected from the group consisting of LiPF₆, LiBF₄, and LiN(SO₂F)₂.

4. An energy storage device comprising:
the non-aqueous electrolytic solution for an energy storage device according to any one of claims 1 to 3;
a positive electrode; and
a negative electrode.

## Amended claims

### Amended claims under Art. 19.1 PCT

1. A non-aqueous electrolytic solution for an energy storage device, comprising:
a non-aqueous solvent;
an electrolyte salt dissolved in the non-aqueous solvent;
a first additive consisting of a sulfolane compound represented by the following formula (1); and
a second additive that is at least one selected from the group consisting of a cyclic sulfuric ester compound represented by the following formula (2a) and a dinitrile compound represented by the following formula (2b): 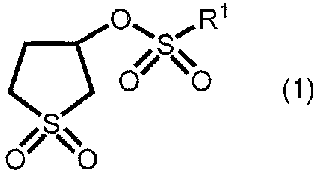 in the formula (1), R¹ represents a methyl group, an ethyl group, a vinyl group, a phenyl group, or a tolyl group, 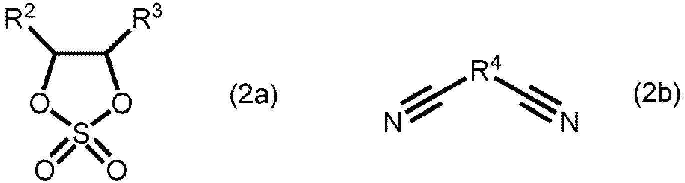
in the formula (2a), R² and R³ each independently represent a methyl group, an ethyl group, or a hydrogen atom, and
in the formula (2b), R⁴ represents an alkylene group having 1 to 6 carbon atoms.

2. The non-aqueous electrolytic solution for an energy storage device according to claim 1, further comprising a third additive formed of at least one lithium salt represented by the following formula (3a), (3b), (3c), (3d), or (3e):

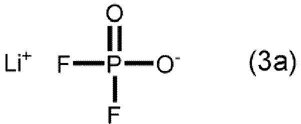

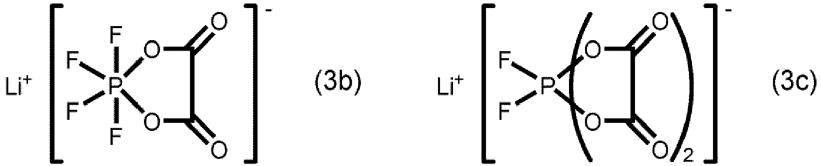

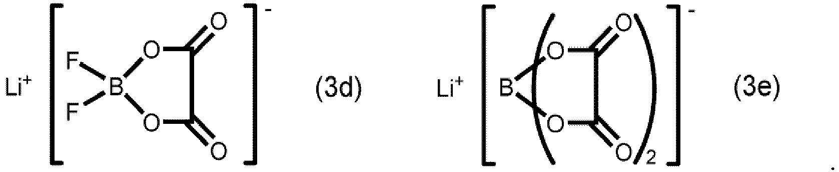

3. The non-aqueous electrolytic solution for an energy storage device according to claim 1 or 2, wherein the electrolyte salt includes at least one lithium salt selected from the group consisting of LiPF₆, LiBF₄, and LiN(SO₂F)₂.

4. (Currently Amended) The non-aqueous electrolytic solution for an energy storage device according to any one of claim 1 to 3, wherein
the content of the first additive is 0.1% by mass or more and 5% by mass or less based on the mass of the non-aqueous electrolytic solution, and
the content of the second additive is 0.1% by mass or more and 5% by mass or less based on the mass of the non-aqueous electrolytic solution.

5. (New) The non-aqueous electrolytic solution for an energy storage device according to any one of claim 1 to 4, wherein the second additive is the cyclic sulfuric ester compound represented by the following formula (2a).

6. (New) An energy storage device comprising:
the non-aqueous electrolytic solution for an energy storage device according to any one of claims 1 to 5;
a positive electrode; and
a negative electrode.
